# EUROPEAN PATENT SPECIFICATION

(11) **EP 2 863 230 B1**
(45) Date of publication and mention of the grant of the patent: **29.11.2017**
(21) Application number: 13306433.7
(22) Date of filing: 18.10.2013
(51) Int. Cl.: G01N 33/68

(54) **A method for predicting the risk of sepsis of a patient undergoing surgery**
Verfahren zur Vorhersage des Risikos fuer Sepsis eines Patienten während eines chirurgischen Eingriffs
Procédé permettant de prédire le risque d'une sepsie chez un patient subissant une intervention chirurgicale

(43) Date of publication of application: 22.04.2015
(73) Proprietor: UNIVERSITE DE BOURGOGNE, 21000 Dijon (FR); INSERM (Institut National de la Santé et de la Recherche Médicale), 75013 Paris (FR); Centre Hospitalier Universitaire De Dijon, 21079 Dijon Cedex (FR)
(72) Inventor: LAGROST, Laurent, 21079 Dijon Cedex (FR); GIRARD, Claude, 21079 Dijon Cedex (FR); BONITHON-KOPP, Claire, 21079 Dijon Cedex (FR)
(74) Representative: Cabinet Plasseraud

(56) References cited:
- EP-A1- 2 339 352
- CINTIA M. C. GRION ET AL: "Lipoproteins and CETP levels as risk factors for severe sepsis in hospitalized patients", EUROPEAN JOURNAL OF CLINICAL INVESTIGATION, vol. 40, no. 4, 1 April 2010 (2010-04-01), pages 330-338, XP55091503, ISSN: 0014-2972, DOI: 10.1111/j.1365-2362.2010.02269.x
- Renana Shor ET AL: "Low Serum LDL Cholesterol Levels and the Risk of Fever, Sepsis, and Malignancy", Annals of Clinical and Laboratory Science, 1 January 2007 (2007-01-01), page 343, XP55091505, United States Retrieved from the Internet: URL:http://www.ncbi.nlm.nih.gov/pubmed/180 00291 [retrieved on 2013-12-04]
- M Anton: "Recent advances concerning the functional properties of egg yolk low-density lipoproteins", , 15 June 2006 (2006-06-15), XP055091655, Retrieved from the Internet: URL:http://www.cabi.org/animalscience/Uplo ads/File/AnimalScience/additionalFiles/WPS AVerona/10903.pdf [retrieved on 2013-12-04]
- DOBESH P P ET AL: "Statins in sepsis", JOURNAL OF PHARMACY PRACTICE, TECHNOMIC PUBLISHING CO, US, vol. 23, no. 1, 1 January 2010 (2010-01-01), pages 38-49, XP009174718, ISSN: 0897-1900, DOI: 10.1177/0897190009356548
- MOKART D ET AL: "Procalcitonin, interleukin 6 and systemic inflammatory response syndrome (SIRS): early markers of postoperative sepsis after major surgery", BRITISH JOURNAL OF ANAESTHESIA, BJM PUBLISHING GROUP, LONDON, GB, vol. 94, no. 6, 1 January 2005 (2005-01-01), pages 767-773, XP007913942, ISSN: 0007-0912, DOI: 10.1093/BJA/AEI143 [retrieved on 2005-04-22]
- Concepclon Alvarez ET AL: "Lipids, Lipoproteins, and Apoproteins in Serum during Infection MaterIals and Methods", CLIN. CHEM. CLINICAL CHEMISTRY, 1 January 1986 (1986-01-01), pages 142-145, XP55091466, Retrieved from the Internet: URL:http://www.clinchem.org/content/32/1/1 42.full.pdf#page=1&view=FitH [retrieved on 2013-12-04]
- A Stachon ET AL: "Prognostic significance of low serum cholesterol after cardiothoracic surgery", Clinical chemistry, 1 August 2000 (2000-08-01), page 1114, XP55091266, UNITED STATES Retrieved from the Internet: URL:http://www.clinchem.org/cgi/content/ab stract/46/8/1114 [retrieved on 2013-12-04]
- SRIVASTAVA L M ET AL: "Evaluation of procalcitonin and hypocholesterolemia in sepsis", CLINICAL CHEMISTRY, AMERICAN ASSOCIATION FOR CLINICAL CHEMISTRY, WASHINGTON, DC, vol. 54, no. 6, Suppl, 1 June 2008 (2008-06-01), page A148, XP009174708, ISSN: 0009-9147
- BILLER KATHARINA ET AL: "Diagnostic and prognostic value of biochemical markers in critically ill patients on ICU", IMFLAMMATION RESEARCH, BIRKHAEUSER VERLAG, BASEL, CH, vol. 59, no. Suppl. 1, 1 May 2010 (2010-05-01), pages S137-S138, XP009174804, ISSN: 1023-3830
- MEMIS ET AL: "High C-reactive protein and low cholesterol levels are prognostic markers of survival in severe sepsis", JOURNAL OF CLINICAL ANESTHESIA, BUTTERWORTH PUBLISHERS, STONEHAM, GB, vol. 19, no. 3, 1 May 2007 (2007-05-01), pages 186-191, XP022093314, ISSN: 0952-8180, DOI: 10.1016/J.JCLINANE.2006.10.008
- CHIEN JUNG-YIEN ET AL: "LOW SERUM LEVEL OF HIGH-DENSITY LIPOPROTEIN CHOLESTEROL IS A POOR PROGNOSTIC FACTOR FOR SEVERE SEPSIS", CRITICAL CARE MEDICINE,, vol. 33, no. 8, 1 August 2005 (2005-08-01) , pages 1688-1693, XP009081612, ISSN: 0090-3493
- VAN LEEUWEN HENK J ET AL: "Lipoprotein metabolism in patients with severe sepsis", CRITICAL CARE MEDICINE,, vol. 31, no. 5, 1 May 2003 (2003-05-01), pages 1359-1366, XP009174781, ISSN: 0090-3493
- FRAUNBERGER P ET AL: "Sepsis-induced hypocholesterolemia", ATHEROSCLEROSIS, ELSEVIER IRELAND LTD, IE, vol. 151, no. 1, 1 July 2000 (2000-07-01), page 320, XP027334983, ISSN: 0021-9150 [retrieved on 2000-07-01]
- A Böning ET AL: "Postoperatives Cholesterin und Erythroblasten als Parameter der perioperativen Sterblichkeit nach herzchirurgischen Eingriffen", Zeitschrift f r Herz-, Thorax- und Gef chirurgie, 1 December 2001 (2001-12-01), pages 242-248, XP55091270, DOI: 10.1007/s003980170002 Retrieved from the Internet: URL:http://download.springer.com/static/pd f/687/art%3A10.1007%2Fs003980170002.pdf?au th66=1386246403_537dacaf2966544ee1d60373d5 97036d&ext=.pdf [retrieved on 2013-12-04]
- TEN BOEKEL E ET AL: "Clinical laboratory findings associated with in-hospital mortality", CLINICA CHIMICA ACTA, ELSEVIER BV, AMSTERDAM, NL, vol. 372, no. 1-2, 1 October 2006 (2006-10-01), pages 1-13, XP027877693, ISSN: 0009-8981 [retrieved on 2006-10-01]
- JECKO THACHIL ET AL: "Lipoproteins, statins and septic haematology patients", BRITISH JOURNAL OF HAEMATOLOGY, vol. 155, no. 1, 12 April 2011 (2011-04-12), pages 122-123, XP55090888, ISSN: 0007-1048, DOI: 10.1111/j.1365-2141.2011.08642.x

## Description

### FIELD OF THE INVENTION

The present invention relates to a method for predicting the risk of sepsis of a patient undergoing surgery.

### BACKGROUND OF THE INVENTION

Systemic inflammatory response syndrome (SIRS) is frequently associated with cardiopulmonary bypass (CPB) that is routinely used during cardiac surgery. Perioperative SIRS can occur through at least two independent mechanisms: i) the contact activation of circulating blood elements as exposed to artificial surfaces in the bypass circuit (1), and ii) the bacterial translocation due to increased gut permeability (2, 3). In the former case, SIRS is only transient and restricted to the operative period thanks to significant improvements of clinical practices and the use of biocompatible CPB circuits (4). In the second case, detectable endotoxemia may occur and complications can extend beyond the perioperative period, leading to a poorer clinical outcome and an increased risk of sepsis (5, 6). Today, sepsis which can develop after cardiac surgery under CPB is largely unpredictable. Whereas most of patients can handle spontaneously the transient inflammatory stress due to CPB, prevention and treatment of CPB-induced sepsis is much more problematic. In this context, identification of independent prognostic factors of sepsis and associated poor clinical outcome is urgently needed. Today, assessment and monitoring of sepsis is mainly based on clinical criteria and score evaluation in the absence of consensual, simple and reliable biological markers.

### SUMMARY OF THE INVENTION

The present disclosure relates to a method for predicting the risk of sepsis for a patient undergoing surgery, comprising the step of measuring preoperative cholesterolemia of said patient, wherein low preoperative cholesterolemia is associated with an increased risk of sepsis.

The present invention relates to a method for predicting sepsis onset in a patient who has undergone a surgical operation and who had a low preoperative cholesterolemia, wherein said method comprises the step of measuring the IL-8 blood level in said patient after the surgical operation, wherein an increase in IL-8 level within 6 hours after the surgical operation is associated with sepsis onset.

The present invention also relates to a method for predicting sepsis onset in a patient who has undergone a surgical operation and who had a low preoperative cholesterolemia, wherein said method comprises the step of measuring the IL-8 and procalcitonin (PCT) blood levels in said patient after the surgical operation, wherein an increase in IL-8 and PCT levels within 6 hours after the surgical operation is associated with sepsis onset.

The present disclosure also relates to a method for diminishing the risk of sepsis of a patient undergoing a surgical operation, who has a low preoperative cholesterolemia, wherein said method comprises the step of administering to said patient before or during the surgical operation an effective amount of an agent increasing the cholesterolemia.
Thus, the present disclosure relates to an agent increasing the cholesterolemia for use in a method for diminishing the risk of sepsis of a patient undergoing a surgical operation, who has a low preoperative cholesterolemia, wherein said agent is administered to said patient before or during the surgical operation.

### DETAILED DESCRIPTION OF THE INVENTION

Typically the surgery is a surgery associated with a postoperative risk of sepsis for the patient.
Bacterial translocation and sepsis frequently occur during a surgical operation, for instance including bowel surgery, lung surgery, cardiac surgery, laparotomy, elective surgery, aortic aneurysm repair, or liver resection, (cf. Dig Surg. 2009;26(2): 100-9. Role of translocation of pathogen-associated molecular patterns in sepsis.Tsujimoto H, Ono S, Mochizuki H).

Examples of a surgery associated with a postoperative risk of sepsis for the patient are bowel surgery, lung surgery, cardiac surgery, laparotomy, elective surgery, aortic aneurysm repair, or liver resection.

In a preferred embodiment, the surgery is bowel surgery, lung surgery, or cardiac surgery.
In a more preferred embodiment, the surgery is cardiac surgery with cardiopulmonary bypass.

Typically the cholesterolemia may be measured by any conventional method.

Typically, to determine whether the cholesterolemia of the patient is low, a comparison will be made with reference value.
Typically, the preoperative cholesterolemia may be determined less than 3, 2 or 1 weeks, or less than 7, 6, 5, 4, 3, 2, 1 days before the surgical operation.
In preferred embodiment, the preoperative cholesterolemia may be determined less than 12 hours before the surgical operation.

Typically, IL-8 and PCT blood levels may be measured by any conventional method, including immunoassays such as ELISA or Cytometric Bead Array for human inflammatory cytokines.

Typically to detect an increase in IL-8 or PCT levels after the surgical operation, IL-8 or PCT levels may be measured just after the end of the surgical operation and within 2,3, 4, 5 or 6 hours after the end of the surgical operation.

Agents able to increase the cholesterolemia of a patient are well known.
Typically the agent may be a lipoprotein composition.
Examples of lipoprotein compositions increasing the cholesterolemia of a patient are native chylomicrons, native very low density lipoproteins (VLDL), intermediate density lipoproteins (IDL), native low density lipoproteins (LDL), native high density lipoproteins (HDL), reconstituted lipoproteins, apolipoprotein AI mimetics, apo A-1 Milano/phospholipid or apo A-1/lipid complex infusion ... (cf. for example Chapman MJ. Comparative analysis of mammalian plasma lipoproteins. Methods Enzymol. 1986;128:70-143 and Chapman MJ. Animal lipoproteins: chemistry, structure, and comparative aspects. J Lipid Res. 1980 Sep;21(7):789-853).

Among established treatments able to modify cholesterolemia are dietary intervention and HDL-raising drugs including statins, fibrates, thiazolidinediones, nicotinic acids, cholesterol ester transfer protein (CETP) inhibitors, liver X receptor (LXR) agonists, apo A-1 Milano/phospholipid or apo A-1/lipid complex infusion (cf. for example Curr Pharm Des. 2010 May;16(13):1517-30. High-density lipoprotein-raising strategies: update 2010. Spillmann F, Schultheiss HP, Tschöpe C, Van Linthout S; Vascular Health and Risk Management 2010:6 979-996 and Pharmacology & Therapeutics 137 (2013) 341-351).

In the following, the invention will be illustrated by means of the following example, figures and tables.

### FIGURE LEGENDS

**Figure 1** **- Time-course curves of cytokine concentrations in patient subgroups.** Plasma concentrations of IL8 (1a), IL6 (1b), TNFalpha (1c) and ILlbeta (1d) were measured in patients without SIRS, with SIRS without sepsis, and with SIRS with sepsis. Both IL8 and IL6 concentrations peaked 3 hours after CPB in sepsis patients, and differences between patients with sepsis and those without sepsis were highly significant (p values <0.005).

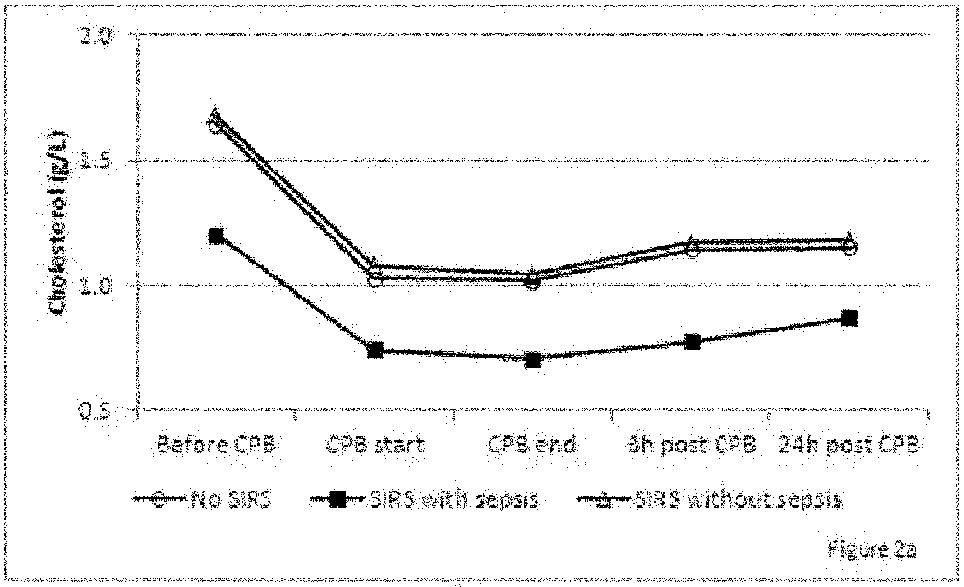

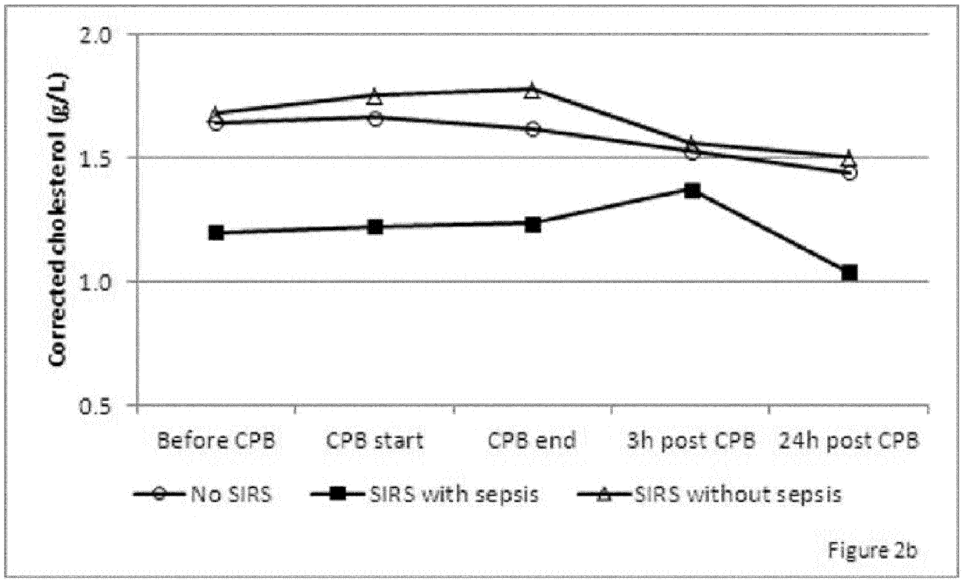

**Figure 2** - **Time-course curves of cholesterol concentrations in patient subgroups.** Plasma cholesterol concentrations in patients without SIRS, with SIRS without sepsis, and with SIRS with sepsis were expressed as absolute values (2a) or as corrected for hemodilution using albuminemia (2b) (see Materials and Methods). In all patient subgroups, changes in absolute cholesterol concentration from baseline values and during the course of operation were statistically significant (p<0.009 in all cases - 2a). When values were corrected for hemodilution, plasma cholesterol was stable during CPB in all subgroups and it decreased significantly 24h after CPB in sepsis patients, and 3h and 24h after CPB in non-SIRS patients and in SIRS patients without sepsis (P<0.01 in all cases - 2b). At any time point, and whether values were corrected or not for hemodilution, plasma cholesterol concentration was constantly and significantly lower in sepsis patients than in patients without sepsis (p<0.001), with the exception of the 3-h, post-CPB time point when there was no significant difference between the three subgroups.
**Figure 3** **- Baseline cholesterol levels in patient subgroups after stratification on surgery, statin use, BMI, diabetes, inflammatory diseases and EuroSCORE.** Baseline cholesterol was consistently lower in sepsis patients than in patients without SIRS and those with SIRS without sepsis.
**Figure 4** **- Receiver-Operator characteristic curve for the prediction of confirmed sepsis using baseline cholesterol**
**Figure 5** **- Baseline cholesterol levels by procalcitonin (PCT) quintiles.**

**Table 1: Baseline clinical characteristics of patients undergoing elective cardiac surgery with cardiopulmonary bypass**

| | All patients | | Non SIRS patients | | SIRS patients without sepsis | | SIRS patients with sepsis | | p value |
|---|---|---|---|---|---|---|---|---|---|
| | n=217 | | n=107 | | n=95 | | n=15 | | |
| Median age [IQR] | 71.7 | [62.1 - 77.1] | 73.3 | [61.2 -78.2] | 67.8 | [61.9 -74.7] | 75.6 | [71.8 - 81.6] | 0.009^{a} |
| Median body mass index [IQR] | 26.4 | [24.4 - 29.4] | 26.0 | [23.8 - 28.6] | 27.1 | [25.0 - 30.5] | 27.3 | [23.7 - 29.3] | 0.037 |
| Male gender n (%) | 148 | (68.2) | 76 | (71.0) | 62 | (65.3) | 10 | (66.7) | 0.67 |
| Surgery | | | | | | | | | |
| Coronary by-pass n (%) | 73 | (33.6) | 37 | (34.6) | 33 | (34.7) | 3 | (20.0) | 0.40 |
| Valve surgery n (%) | 104 | (47.9) | 55 | (51.4) | 41 | (43.2) | 8 | (53.3) | |
| Both n (%) | 40 | (18.4) | 15 | (14.0) | 21 | (22.1) | 4 | (26.7) | |
| Median left ventricular ejection fraction [IQR] | 60.0 | [52.0 - 68.0] | 62.0 | [56.0 - 68.0] | 60.0 | [50.0 - 68.0 | 59.0 | [53.0 - 69.0] | 0.34 |
| Median EuroSCORE [IQR] | 6.0 | [3.0-8.0] | 6.0 | [3.0-8.0] | 6.0 | [3.0-8.0] | 9.0 | [5.0-10.0] | 0.019 |
| Personal history | | | | | | | | | |
| Atrial fibrillation (%) | 44 | (20.3) | 18 | (16.8) | 21 | (22.1) | 5 | (33.3) | 0.28 |
| Hypertension (%) | 153 | (70.5) | 72 | (67.3) | 69 | (72.6) | 12 | (80.0) | 0.50 |
| Diabetes (%) | 46 | (21.2) | 18 | (16.8) | 21 | (22.1) | 7 | (46.7) | 0.029^{b} |
| Dyslipidemia (%) | 136 | (62.7) | 63 | (58.9) | 61 | (64.2) | 12 | (80.0) | 0.26 |
| Statin use (%) | 123 | (56.7) | 56 | (52.3) | 56 | (59.0) | 11 | (73.3) | 0.26 |
| Smoking (%) | 51 | (23.5) | 25 | (23.4) | 24 | (25.3) | 2 | (13.3) | 0.60 |
| Inflammatory disease^{c} (%) | 44 | (20.3) | 15 | (14.0) | 23 | (24.2) | 6 | (40) | 0.029 ^{d} |
| Renal failure (%) | 16 | (7.4) | 7 | (6.5) | 6 | (6.3) | 3 | (20) | 0.15 |

| | | | | | | | | | |
|---|---|---|---|---|---|---|---|---|---|
| ^{a} Between-category comparisons: sepsis *versus* non SIRS patients: p=0.12; sepsis *versus* SIRS patients without sepsis: p=0.008 ^{b} Between- category comparisons: sepsis *versus* non SIRS patients: p=0.014; sepsis *versus* SIRS patients without sepsis: p=0.057 ^{c} chronic inflammatory disease includes arthritis (n=23),chronic obstructive pulmonary disease (n=10), asthma (n=9), chronic pancreatitis (n=1) and temporal arteritis (n=1). ^{d} Between-category comparisons: sepsis *versus* non SIRS patients: p=0.023; sepsis *versus* SIRS patients without sepsis: p=0.22 | | | | | | | | | |

**Table 2: Operation duration, and markers of acute kidney injury and cardiac output in patients undergoing cardiac surgery with cardiopulmonary bypass (CPB)**

| | Non SIRS patients | | SIRS patients without sepsis | | SIRS patients with sepsis | | P value |
|---|---|---|---|---|---|---|---|
| | (n=107) | | (n=95) | | (n=15) | | |
| | Median | [IQR] | Median | | Median | [IQR] | |
| Aortic cross-clamp time (min) | 64.0 | [45.0 - 87.0] | 70.0 | [51.0 - 88.0] | 81.0 | [62.0 - 110.0] | 0.08 |
| CPB time (min) | 92.0 | [69.0 - 113.0] | 93.0] | [73.0 - 128.0] | 129 | [104- 178] | 0.01^{b} |
| 3h-post CPB NGAL (ng/ml) | 411^{a} | [311 - 518] | 451] | [344 - 581] | 699 | [494 - 1331] | < 0.001^{c} |
| 3h-post CPB NT-proBNP (pg/ml) | 644^{a} | [329 - 1838] | 585 | [232 - 1478] | 1156 | [469 - 4143] | 0.057 |

| | | | | | | | |
|---|---|---|---|---|---|---|---|
| ^{a} Missing biological samples for 3 patients ^{b} Between category comparisons: sepsis *versus* non SIRS patients: p=0.004; sepsis *versus* SIRS patients without sepsis: p=0.010 ^{c} Between category comparisons: sepsis *versus* non SIRS patients: p<0.001; sepsis *versus* SIRS patients without sepsis: p=0.001 | | | | | | | |

**Table 3: Baseline plasma lipid and albumin levels in patient subgroups**

| | Non SIRS patients (1) | | SIRS patients without sepsis (2) | | SIRS patients with sepsis (3) | | p | p | p |
|---|---|---|---|---|---|---|---|---|---|
| | n=107 | | n=95 | | n=15 | | | | |
| | Median | [IQR] | Median | [IQR] | Median | [IQR] | | (1) vs (3) | (2) vs (3) |
| Triglycerides (g/L) | 1.14 | [0.88 - 1.46] | 1.41 | [1.12 - 1.87] | 1.12 | [0.88 - 1.50] | 0.0001 | 0.6463 | 0.0091 |
| Total cholesterol | 1.64 | [1.37 - 1.92] | 1.68 | [1.34 - 2.04] | 1.20 | [1.06 - 1.47] | 0.0010 | 0.0005 | 0.0008 |
| Albumin (g/L) | 36.1 | [34.1- 38.1] | 35.7 | [33.8 - 38.6] | 36.2 | [35.5-38.6] | 0.42 | | |

**Table 4 - Main complications and therapeutic strategies after cardiopulmonary by-pass surgery by quintiles of baseline cholesterol levels**

| | Q1 cholesterol < 1.23 g/L | Q2 cholesterol [1.23-1.55 g/L[ | Q3 cholesterol [1.55-1.74 g/L[ | Q4 cholesterol [1.74 - 2.03[ g/L) | Q5 cholesterol >= 2.03 g/L | P |
|---|---|---|---|---|---|---|
| | n=43 | n=43 | n=44 | n=43 | n=44 | |
| Median Intubation duration (h) | 12.6 | 12.4 | 10.3 | 10.6 | 9.8 | 0.049 |
| | [9.2-25.5] | [9.3-19.9] | [8.5-16.1] | [8.7-13.6] | [8.1-13.6] | |
| | 28.6 | 14.6 | 13.6 | 4.7 | 9.1 | 0.022 |
| Intubation duration>24h (%) | | | | | | |
| Noradrenaline use (%) | 60.7 | 53.5 | 59.1 | 48.8 | 29.6 | 0.028 |
| *Intra-aortic balloon* counter pulsation (%) | 9.3 | 7.0 | 6.8 | 2.3 | 0 | 0.22 |
| Dialysis (%) | 4.7 | 7.0 | 0 | 0 | 0 | 0.048 |
| Blood transfusion (%) | 51.2 | 46.5 | 47.7 | 41.9 | 18.2 | 0.013 |
| Atrial fibrillation^{a} (%) | 20.9 | 18.6 | 22.7 | 18.6 | 18.2 | 0.98 |
| Renal failure^{a} (%) | 23.3 | 27.9 | 29.6 | 25.6 | 22.7 | 0.94 |
| Sepsis (%) | 18.6 | 9.3 | 4.6 | 2.3 | 0 | 0.005 |

| | | | | | | |
|---|---|---|---|---|---|---|
| ^{a} New occurrence of atrial fibrillation or renal failure after cardiopulmonary by-pass surgery | | | | | | |

**Table 5: Multivariate analysis of factors associated with high procalcitonin (PCT), high IL6 , or high IL8 levels measured 3 hours after CPB (n = 211)**

| | **Dependent variable ^{a} PCT^{b}** | | | **Dependent variable ^{a} IL8^{c}** | | | **Dependent variable ^{a} IL6^{d}** | | |
|---|---|---|---|---|---|---|---|---|---|
| | OR | 95% CI | P | OR | 95% CI | P | OR | 95% CI | P |
| Baseline cholesterol (for an increase of 0.40 g/L) | 0.59 | [0.38 - 0.90] | 0.014 | 0.56 | [0.37 - 0.86] | 0.008 | 0.80 | [0.54 - 1.17] | 0.25 |
| Age | 0.99 | [0.94 - 1.05] | 0.82 | 0.98 | [0.93 - 1.03] | 0.34 | 0.98 | [0.93 - 1.03] | 0.33 |
| Female gender | 0.93 | [0.37 - 2.35] | 0.88 | 4.44 | [1.86 - 10.6] | <0.001 | 1.00 | [0.43 - 2.37] | 0.99 |
| Surgery | | | | | | | | | |
| Coronary bypass | 1 | | | 1 | | | 1 | | |
| Valve surgery | 0.59 | [0.19 - 1.79] | 0.35 | 0.96 | [0.32 - 2.88] | 0.95 | 0.88 | [0.29 - 2.67] | 0.83 |
| Both | 0.59 | [0.176-2.15] | 0.42 | 0.95 | [0.26 - 3.51] | 0.94 | 1.73 | [0.50- 5.96] | 0.38 |
| Atrial fibrillation | 2.28 | [0.86 - 6.02] | 0.097 | 2.06 | [0.74 - 5.73] | 0.17 | 3.08 | [1.23 - 7.68] | 0.016 |
| Hypertension | 1.33 | [0.50 -3.56] | 0.57 | 1.13 | [0.42 - 3.03] | 0.80 | 1.77 | [0.66- 4.75] | 0.26 |
| Dyslipidemia | 1.10 | [0.43 - 2.79] | 0.84 | 2.36 | [0.90 - 6.22] | 0.08 | 1.06 | [0.44 - 2.54] | 0.90 |
| EuroSCORE | 1.04 | [0.90- 1.20] | 0.57 | 1.17 | [1.01-1.35] | 0.033 | 1.19 | [1.04 - 1.38] | 0.013 |
| CPB time (for an increase of 10 min) | 1.34 | [1.19 - 1.52] | < 0.001 | 1.21 | [1.09 - 1.36] | <0.001 | 1.17 | [1.05- 1.29] | 0.003 |

| | | | | | | | | | |
|---|---|---|---|---|---|---|---|---|---|
| ^{a} logistic regression models including as independent variables all factors associated with either high PCT levels or IL8 levels or IL6 levels with a p value <0.10 in univariate analysis. ^{b} High procalcitonin levels defined as levels ≥ 0.37 ng/mL (top quintile of the distribution) ^{c} High IL8 levels defined as levels ≥ 264 pg/mL (top quintile of the distribution) ^{d} High IL6 levels defined as levels ≥ 733 pg/mL (top quintile of the distribution) | | | | | | | | | |

### EXAMPLE

*Objective:* Systemic inflammatory response syndrome (SIRS) and sepsis frequently occur after cardiac surgery with cardiopulmonary bypass (CPB). The aim of the present study was to investigate whether preoperative cholesterolemia can predict sepsis onset and postoperative complications in patients undergoing cardiac surgery with CPB.
*Design:* Prospective observational study
*Setting:* Surgical intensive care unit of a French university hospital.
*Patients:* 217 consecutive patients older than 18 years admitted for planned cardiac surgery with CPB.
*Interventions:* Measurements of plasma blood lipids and inflammation markers before anesthesia induction (baseline), at CPB start, at CPB end and 3-hours and 24-hours after cardiac surgery. Values and clinical outcome were compared in SIRS patients with confirmed sepsis (n=15), SIRS patients without confirmed sepsis (n=95) and non-SIRS patients (n=107).
*Measurements and Main Results:* Gradual decrease in plasma cholesterol concentration occured during surgery with CPB, but it did no longer appear after correction for hemodilution. Corrected cholesterolemia was significantly lower at baseline in sepsis patients than in other subgroups, and it remained lower in the sepsis group during and after CPB. With regard to sepsis, the discriminatory power of baseline cholesterol was fairly good as indicated by receiver operating characteristic curve analysis (area under the curve: 0.78, 95% CI: 0.72-0.84). The frequency of sepsis progressively decreased by baseline cholesterolemia quintiles (18.6% and 0% in the bottom and top quintiles respectively, p=0.005). Plasma levels of NGAL, NT-proBNP, PCT, IL6 and IL8 measured 3 hours after CPB were significantly higher in patients with sepsis compared to other subgroups. In multivariate analysis, baseline cholesterolemia and CPB duration were significant and independent determinants of 3 hour-post CPB elevation of PCT and IL8, but not of IL6 concentration.
*Conclusions:* Low cholesterolemia before elective cardiac surgery with CPB is a simple biomarker relevant to the early discrimination of patients at high risk of confirmed sepsis.

### MATERIALS AND METHODS

### Patients and data collection

The study protocol was reviewed and approved by the regional Ethics Committee (Comité de Protection des Personnes Est 1, Dijon, France). Two-hundred fifty consecutive patients older than 18 years who were admitted in the university hospital of Dijon (Burgundy, France) for a planned cardiac surgery requiring CPB were enrolled in the study from March 2008 through February 2009. All patients provided signed informed consent during the pre-operative anesthesiologist's consultation. The study protocol planned repeated blood sampling at various times: at entry in the surgery room (*i.e.* before anesthesia induction-baseline), at the start of CPB, at the end of CPB, and 3 and 24 hours after CPB. Fasting blood samples were processed within one hour and plasmas were stored at -80°C until analysis. Thirty three patients were secondarily excluded (4 patients without CPB, 3 deaths during or just after CPB, and 26 patients with incomplete blood sampling) leading to a study sample of 217 patients available for analysis.

The following pre-operative clinical data were collected during the anesthesiologist's consultation: demographic and anthropometric data, left ventricular ejection fraction, co-morbidities and cardiovascular risk factors (atrial fibrillation, hypertension, renal failure, diabetes, obesity, dyslipidemia, smoking, and history of inflammatory disease, redux/tridux, infectious endocarditis or sepsis), current use of medications (β-blockers, statins, diuretics, anti-hypertensive agents, antiarrythmic drugs, platelet suppressive agents, aspirin, non steroidal anti-inflammatory drugs, and anticoagulants). The intraoperative variables recorded included the type of surgery (coronary artery bypass grafting, valvular heart surgery, or both), CPB time, aortic cross clamp time and amount of specific drugs administered during CPB (antifibrinolytic agents, heparin/protamin). The postoperative variables observed included post-CPB need for inotropes, hemofiltration or dialysis, blood transfusion, duration of mechanical ventilation and inotrope usage, and ICU stay. Post-operative complications were recorded.

The primary outcome was the occurrence of SIRS defined by at least two of the following criteria: temperature >38°C or <36°C, heart rate >90 beats/minute, respiratory rate >20 breaths/minute or PaCO2<32 mmHg, white blood cell count >12000 or <4000 cells/mm³. Confirmed sepsis was defined by documented infection with positive culture samples. Other recorded complications were stroke, acute renal failure (creatinine>150 µmol/L or 40% increase from baseline level), new occurrence of atrial fibrillation and hemodynamic complications. Secondary outcomes were the elevation (defined as values in the highest quartile) 3 hours after CPB of procalcitonine (PCT), a known marker of infection and sepsis, and of two inflammatory markers, interleukine-8 (IL8) and IL6.

### Biological analyses

Lipids (total cholesterol and triglycerides) were assayed on a Konelab Prime 301 diagnostic system (Thermo Scientific) using reagents of the manufacturer. Quantitation of cytokines (IL1β, IL6, IL8, and TNFα) was performed on a Guava EasyCyte Plus Flow Cytometry System (Millipore) using the Cytometric Bead Array (CBA) human inflammatory cytokines kit assay from BD Biosciences (US kit number 877.232.8995). PCT levels were measured by a homogeneous one-step sandwich immunoassay using time-resolved amplified cryptate emission technology on Kryptor® system (Thermo-scientific, Asniere, France). Albumin was assayed using a colorimetric method (bromocresol purple) on a Dimension Vista® system (Siemens, Erlangen, Germany). N-terminal pro-brain natriuretic peptide (NT-proBNP) levels were determined by a one-step chemiluminescent immunoassay on a Dimension Vista system (Siemens).

Because cardiac surgery with CPB induces hemodilution, biological parameters during and after CPB were corrected for hemodilution by using plasma albumin as a marker, and in a similar way as it was proposed earlier by using hematocrit (13). Values were corrected by multiplying the observed values at one given time by the ratio between albumin at baseline and albumin at the same time point.

### Statistical analysis

Categorical data are presented as percentages and were compared using chi-square test or fisher exact tests, if appropriate. Continuous variables are presented as medians and interquartile range and were compared using Wilcoxon tests or Kruskall-Wallis tests. Changes over time from baseline value of continuous variables were tested using Wilcoxon test for paired data. The predictive ability of baseline cholesterol with regard to confirmed sepsis was evaluated using Receiver Operating Characteristic (ROC) curve analysis. The area under the curve (AUC) and 95% confidence interval were used to assess the discriminatory power of cholesterol with an AUC of 1 considered perfect and 0.5 considered equal to chance. The highest quartile of PCT, IL-6 and IL-8 measured 3 hours after the end of CPB were considered as surrogate indicators of the risk of sepsis. Multivariable logistic regression models were used to assess the association between baseline cholesterol and these surrogate markers (highest quintile versus the 4 lowest quintiles). All variables associated with either of surrogate markers with a p value<0.10 in univariate analysis were introduced in the models, where age and gender were systematically forced. For all testings, p<0.05 was considered significant. All analyses were performed using STATA software version 11.0 (Stata corporation, College station, Tx, USA).

### RESULTS

### Characteristics of patient subgroups with or without sepsis

A total of 217 patients who underwent elective cardiac surgery with CPB and had complete biological assessment were retained for analysis. Whereas none of patients had clinical evidence of SIRS or infection at baseline, 110 patients developed SIRS after cardiac surgery with CPB, and among them, 15 cases developed confirmed sepsis. As shown in Table 1, patients with sepsis were older than patients with SIRS alone, without any difference with patients without SIRS. No significant differences in BMI, gender, cardiac output or frequency of atrial fibrillation were observed between the 3 groups. History of hypertension, dyslipidemia, smoking, and renal failure did not differ between the 3 groups of patients. History of diabetes or inflammatory disease was significantly more frequent in sepsis as compared to non-SIRS patients. No significant differences in medications, including especially hypolipidemic drugs, were observed between the 3 groups of patients.

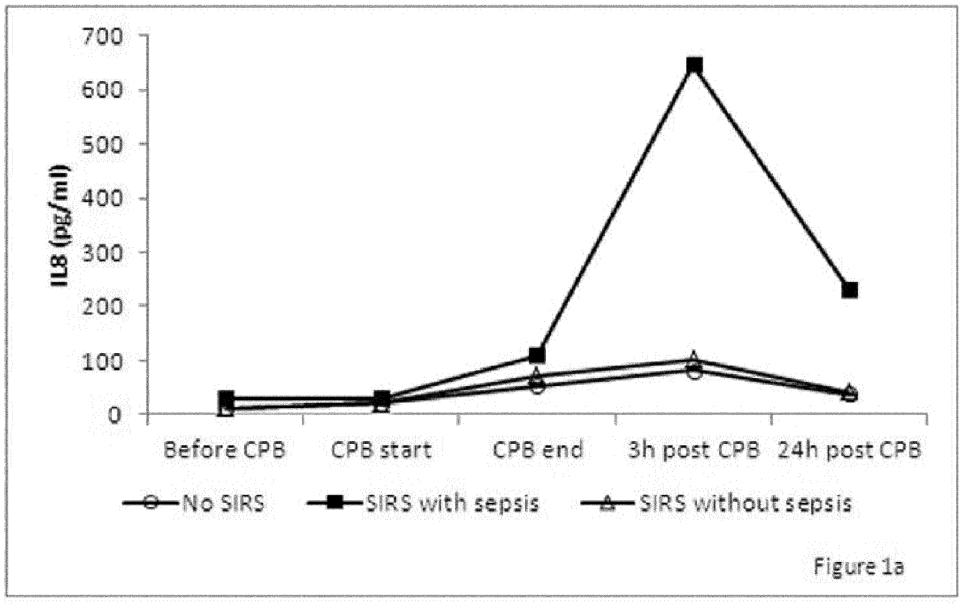

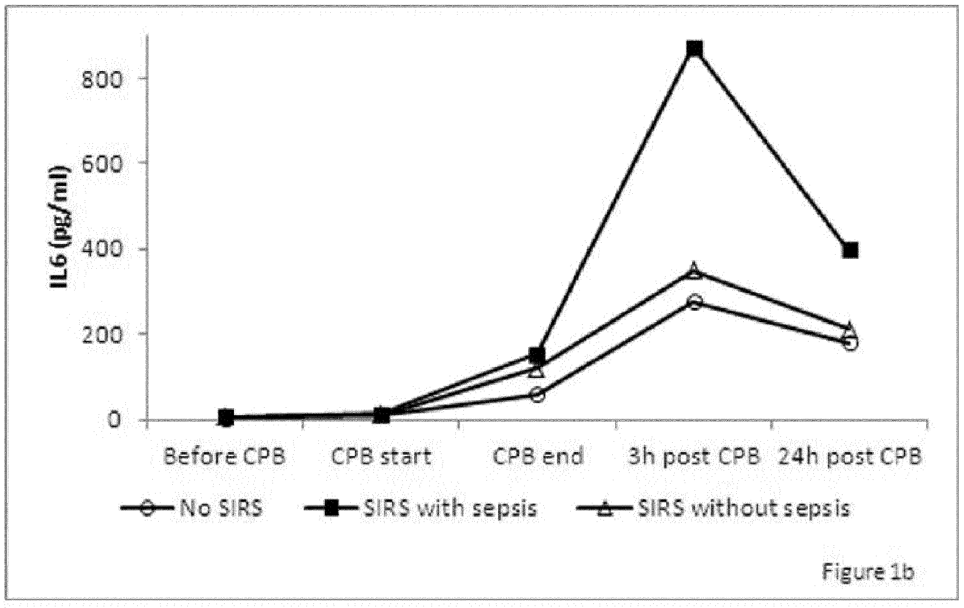

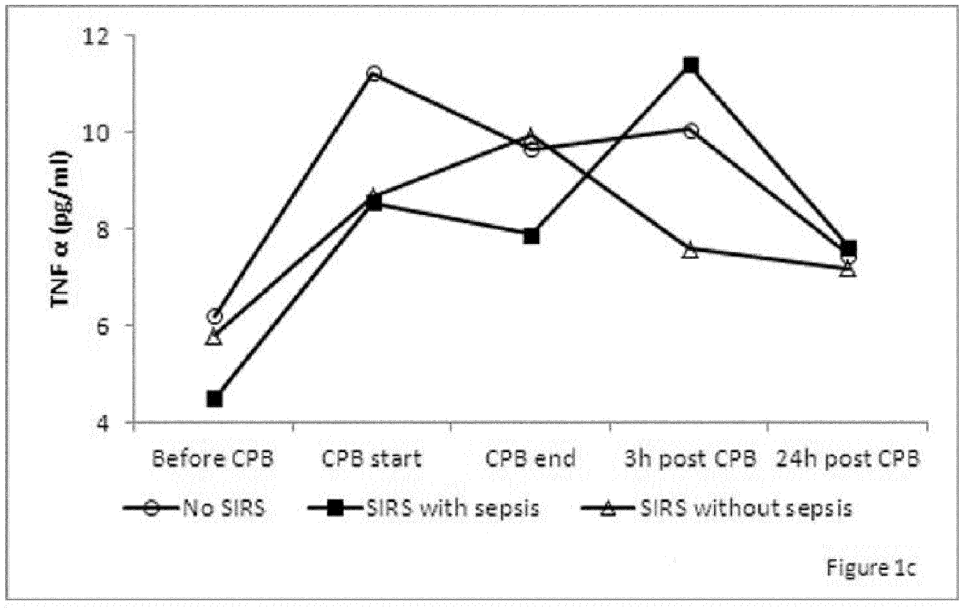

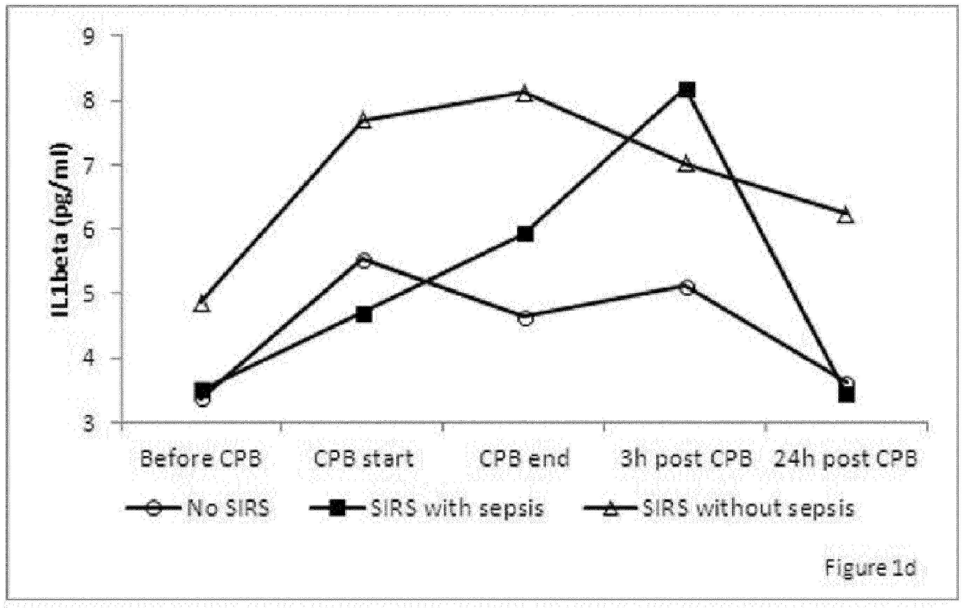

As far as operation duration and markers of kidney injury and perioperative inflammation are concerned, patients with sepsis differed significantly from the others. As shown in Table 2, the median of aortic cross clamp time was significantly higher in sepsis as compared to non-sepsis patients, and more strikingly the median CPB time was markedly longer in sepsis patients as compared to non-SIRS and SIRS patients without sepsis (+40.2 % and +38.7 %, respectively). Plasma levels of both Neutrophil gelatinase-associated lipocalin (NGAL) (*i.e.* an early marker of acute kidney injury after CPB - 14) and N-terminal pro brain natriuretic peptide (NT-proBNP) (*i.e.* an early marker of ventricular dysfunction and atrial fibrillation - 15, 16) were measured 3 hours after CPB. Levels of both markers were significantly higher in sepsis as compared to SIRS patients without sepsis, while they were remarkably similar in non-SIRS and SIRS patients without sepsis (Table 2). Finally, and as shown in Figure 1, plasma concentrations of IL8 and IL6 increased dramatically after CPB to reach maximal values 3 hours after CPB and to decrease 24 hours after CPB. Time-course curves of plasma IL6 and IL8 concentrations were found to differ markedly between patients with or without sepsis, in particular with maximal, 3hr-postCPB values of IL6 and IL8 concentrations that were significantly higher in sepsis than in non-SIRS patients and SIRS patients without sepsis (Figure 1). Unlike IL6 and IL8, neither TNFα, nor IL1β concentrations, as measured before, during and after CPB could discriminate patients with or without sepsis.

### Low plasma cholesterol concentration at baseline: a risk factor of sepsis and poor clinical outcome

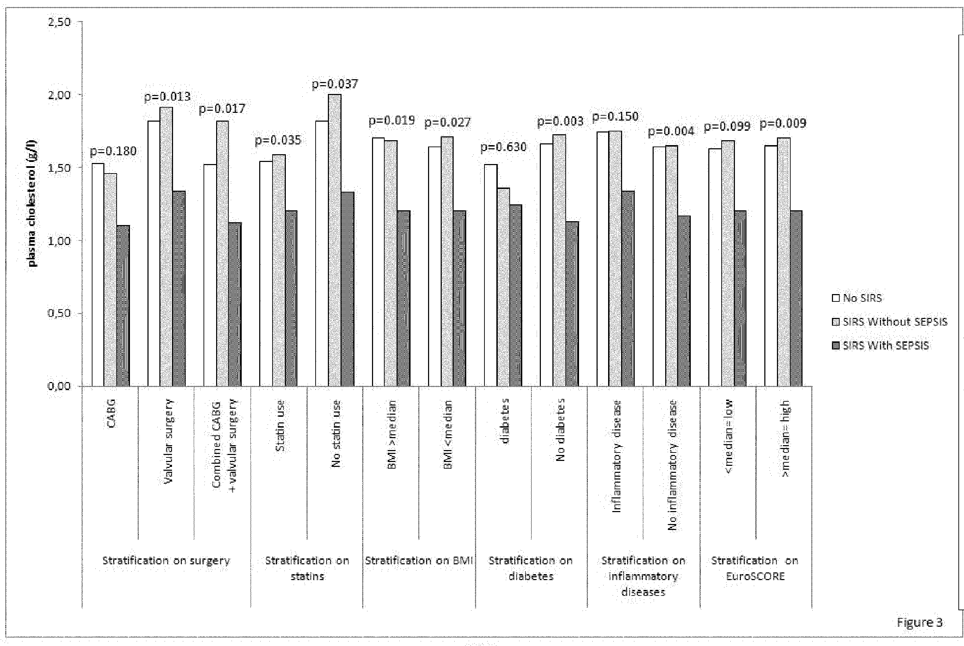

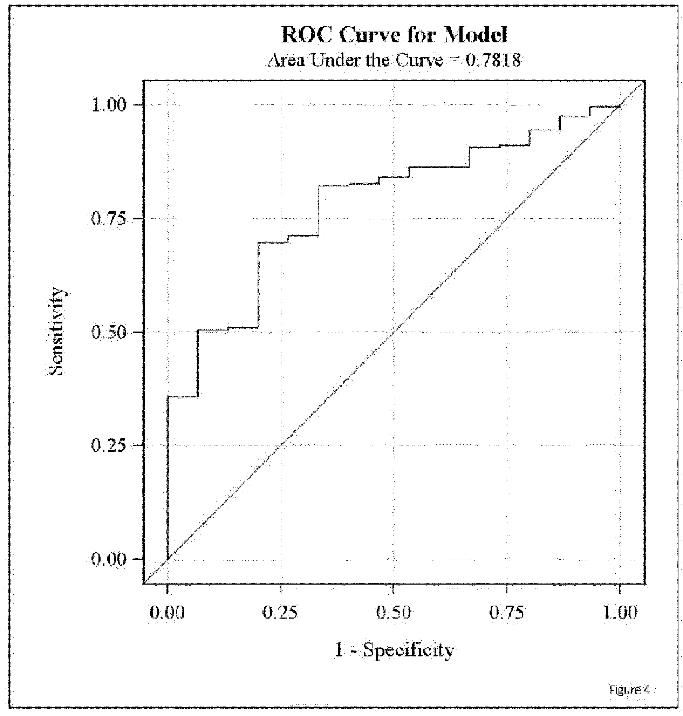

Median preoperative triglyceridemia was significantly lower in patients with sepsis than in SIRS patients without sepsis, but it did not differ from values in patients without SIRS (Table 3). Preoperative total cholesterol level was significantly lower in sepsis patients as compared to both non-SIRS patients and SIRS patients without sepsis. In all subgroups, cholesterolemia was found to decrease gradually during surgery under CPB (Figure 2 - left panel). Time-course curves of cholesterolemia were of similar shape in all three subgroups, but plasma cholesterol values remained constantly lower in sepsis than in non-sepsis subgroups. When corrected for hemodilution by using albuminemia (Figure 2 - right panel), time-dependent decreases in cholesterolemia did no longer appear in any group in the course of CPB. However, and as for significant differences at baseline, corrected plasma cholesterol values during CPB remained systematically lower in sepsis patients as compared to non-SIRS and SIRS patients without sepsis (Figure 2 - right panel). Twenty-four hours after CPB, corrected cholesterolemia tended to decrease in all patient subgroups, again with lower concentration values at the 24-hr, post-CPB time point in sepsis patients (Figure 2 - right panel). When analysis was stratified on various factors (i.e. diabetes, statin use, BMI, type of surgery, inflammatory disease and EuroSCORE), baseline cholesterol levels were consistently lower in patients with sepsis than in those without sepsis (Figure 3). As shown in Figure 4, the ROC analysis revealed that the discriminative power of baseline cholesterol with regard to sepsis was fairly good as assessed by an area under the curve of 0.78 (95% confidence interval: 0.72-0.84). Finally, and in addition to the higher propensity to develop sepsis, low plasma cholesterol level at baseline was found to be associated with more complications and aggressive therapeutic strategies. It is underlined by shorter intubation time, as well as less frequent uses of noradrenaline infusion, dialysis and blood transfusion along quintiles of preoperative cholesterolemia (Table 4).

### Relationship of baseline cholesterol concentration with postoperative PCT, IL6 and IL8 concentrations

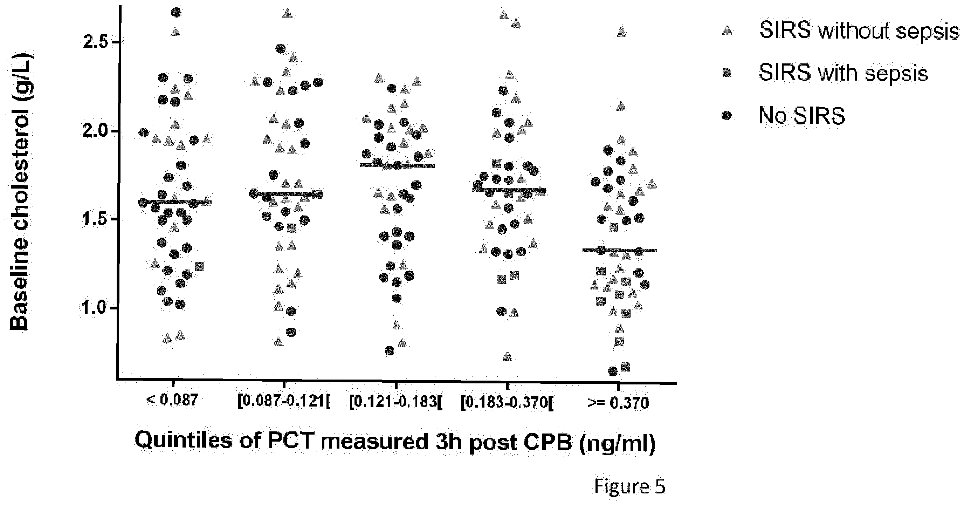

Because plasma concentrations of PCT, IL6 and IL8 were reported to be markedly increased in critically ill patients with suspected sepsis (17), these biological parameters were measured in the present study 3 hours after CPB and used as quantitative biological indicators of sepsis in multivariable analysis. As shown in Figure 5, median total plasma cholesterol was significantly lower in the highest PCT quintile as compared to the others. Interestingly, the highest PCT quintile concentrated most of sepsis patients which contributed to the low median cholesterol value (P=0.007; Figure 5). In multivariable analysis, baseline cholesterol, along with CPB time, was found to constitute an independent and highly significant determinant of PCT and IL8 production, in addition to female gender for IL8. In contrast to PCT and IL8, baseline cholesterol was not an independent predictor of IL6 elevation, which was only related to CPB time, and to a lesser extent to atrial fibrillation (Table 5).

### DISCUSSION

The present study demonstrates that a low preoperative cholesterolemia is associated with an increased risk of sepsis and complications in patients undergoing cardiac surgery with CPB. Furthermore, low baseline cholesterolemia is shown to constitute an independent determinant of two inflammatory markers measured 3 hours after CPB, *i.e.* PCT which is known as a diagnostic parameter of infection and sepsis (18), and IL-8 which is more potent than IL6 to discriminate patients with complications after CPB (13).

Because of deliberate hemodilution during CPB (19), plasma albumin concentration was used in the present study to correct for hemodilution and to standardize plasma cholesterol concentration values. Unlike absolute values, corrected cholesterol concentration values were stable during operation, and tended to decrease 3 hours after CPB was terminated, thus coming in support of a dilutional effect in the course of CPB. Lower baseline cholesterolemia observed in sepsis patients before hemodilution is in agreement with hypocholesterolemia as an independent predictor of clinical outcome in critically ill patients (11). The present study now indicates that low cholesterolemia is a preexisting and persistent risk factor, rather than the consequence of infection and acute phase response in these patients. It brings new and now direct support to the inverse relationship between total cholesterol and the incidence of in-hospital infections that was reported in previous retrospective analyses (20). Importantly, the relationship between low cholesterolemia and a high propensity to develop sepsis was found to be independent of statin treatment which did not differ significantly in the present study between patient subgroups with or without sepsis. Thus, and as highlighted earlier in critically ill surgical patients (11), an adverse effect of lipid-lowering medication could be excluded. On the contrary, statin treatment was reported earlier to decrease the rate of severe sepsis (21), and preoperative statin treatment was associated with fewer postoperative infections after cardiac surgery (22). It suggests that, in case of statins, a putative harmful effect of decreased cholesterolemia might be overcome by concomitant beneficial properties. In particular, it might include reduced bacterial growth as a result of depletion of intracellular stores of cholesterol and precursors (22), or pleiotropic anti-inflammatory and immunomodulating properties (23-25).

Preoperative cholesterolemia was found in the present study to relate inversely to the plasma concentration of PCT measured 3 hours after CPB. We turned towards PCT because it has been reported to constitute a reliable diagnostic parameter for infections and sepsis (18), and to increase selectively during CPB (26). In multivariate analysis, the significant relationship of high PCT with low cholesterolemia was maintained when taking into account other potentially confounding variables, including gender, surgical procedure, atrial fibrillation, renal failure, and beta-blocker medication in addition to CPB duration which is known (27) and confirmed here as an independent risk factor when exceeding 2 hours. In the present study, multivariate analysis revealed that cholesterolemia at baseline was an independent determinant of both PCT and IL8 concentrations measured 3 hours after CPB, whereas no significant relationship was observed with IL6. The lack of relationship with IL6 seems to be in contrast with an earlier study in which plasma lipid levels were found to attenuate the production of IL6 in a small group of patients during CPB (28). However, this latter study did not provide any insights into the clinical outcome of the patients. More recently, the rise of IL8 levels immediately after CPB, unlike IL6, TNFalpha or ILlbeta was significantly and positively associated with duration of postoperative ventilation as an indicator of poor clinical outcome (13). Finally, PCT was proposed to be of higher prognostic value as compared to IL6 and IL8 to identify critically ill patients with sepsis (17), and IL8, but not IL6, was proposed as an independent predictor of complications following cardiac surgery with CPB (29). It is in agreement with the present observations which demonstrate now that, in supplement to clinical diagnosis of sepsis, the combination of a pre-operative low cholesterolemia with post-CPB elevation of PCT and IL8 concentrations should be considered as a strong indicator of infection.

### CONCLUSIONS

The present study brings to the fore low preexisting cholesterolemia as a practicable parameter to discriminate high and low risk patients on preoperative workup. It indicates that correction of low cholesterol levels by infusion of exogenous lipoprotein preparations (as documented earlier in animal models - 9) constitutes a promising and feasible approach in the prevention and treatment of sepsis. Finally, low plasma cholesterol at baseline, in combination with transient increases in PCT and IL8 levels immediately after CPB is of high added value to ascertain diagnosis of infection at an early time point, to predict unfavorable clinical evolution, and to set up appropriate clinical management.

### REFERENCES

Throughout this application, various references describe the state of the art to which the invention pertains.
1 - Day JR, Taylor KM: The systemic inflammatory response syndrome and cardiopulmonary bypass. Int J Surg. 2005, 3:129-40.
2 - Martinez-Pellús AE, Merino P, Bru M, et al: Can selective digestive decontamination avoid the endotoxemia and cytokine activation promoted by cardiopulmonary bypass? Crit Care Med. 1993, 21:1684-91.
3 - Jansen NJ, van Oeveren W, Gu YJ, et al: Endotoxin release and tumor necrosis factor formation during cardiopulmonary bypass. Ann Thorac Surg. 1992, 54:744-7.
4 - Shann KG, Likosky DS, Murkin JM, et al: An evidence-based review of the practice of cardiopulmonary bypass in adults: a focus on neurologic injury, glycemic control, hemodilution, and the inflammatory response. J Thorac Cardiovasc Surg. 2006, 132:283-90.
5 - Rocke DA, Gaffin SL, Wells MT, et al: Endotoxemia associated with cardiopulmonary bypass. J Thorac Cardiovasc Surg. 1987, 93:832-7.
6 - Klein DJ, Briet F, Nisenbaum R, et al: Endotoxemia related to cardiopulmonary bypass is associated with increased risk of infection after cardiac surgery: a prospective observational study. Crit Care. 2011, 15:R69.
7 - Netea MG, Demacker PN, Kullberg BJ, et al: Low-density lipoprotein receptor-deficient mice are protected against lethal endotoxemia and severe gram-negative infections. J Clin Invest. 1996, 97:1366-72.
8 - Chien JY, Jerng JS, Yu CJ, et al: Low serum level of high-density lipoprotein cholesterol is a poor prognostic factor for severe sepsis. Crit Care Med. 2005, 33:1688-93.
9 - Rauchhaus M, Coats AJ, Anker SD. The endotoxin-lipoprotein hypothesis. Lancet. 2000, 356:930-3.
10 - Kalantar-Zadeh K, Block G, Horwich T, et al: Reverse epidemiology of conventional cardiovascular risk factors in patients with chronic heart failure. J Am Coll Cardiol. 2004, 43:1439-44.
11 - Gordon BR, Parker TS, Levine DM, et al: Relationship of hypolipidemia to cytokine concentrations and outcomes in critically ill surgical patients. Crit Care Med. 2001, 29:1563-8.
12 - Rocke DA, Gaffin SL, Wells MT, et al: Endotoxemia associated with cardiopulmonary bypass. J Thorac Cardiovasc Surg. 1987, 93:832-7.
13 - Rothenburger M, Soeparwata R, Deng MC, et al: The impact of anti-endotoxin core antibodies on endotoxin and cytokine release and ventilation time after cardiac surgery. J Am Coll Cardiol. 2001, 38:124-30.
14 - Mishra J, Dent C, Tarabishi R, et al: Neutrophil gelatinase-associated lipocalin (NGAL) as a biomarker for acute renal injury after cardiac surgery. Lancet. 2005, 365:1231-8.
15 - Di Stefano S, Casquero E, Bustamante R, et al: Analysis of inflammatory response and utility of N-terminal pro brain-type natriuretic peptide in cardiac surgery with extracorporeal circulation. J Cardiovasc Med (Hagerstown). 2008, 9:555-60.
16 - Samy K, Anouar J, Mnif E, et al: N-terminal pro-brain natriuretic peptide identifies patients at risk for occurence of postoperative atrial fibrillation in cardiac surgery with cardiopulmonary bypass. Ann Card Anaesth. 2012, 15:199-205.
17 - Harbarth S, Holeckova K, Froidevaux C, et al: Diagnostic value of procalcitonin, interleukin-6, and interleukin-8 in critically ill patients admitted with suspected sepsis. Am J Respir Crit Care Med. 2001, 164:396-402.
18 - Uzzan B, Cohen R, Nicolas P, et al: Procalcitonin as a diagnostic test for sepsis in critically ill adults and after surgery or trauma: a systematic review and meta-analysis. Crit Care Med. 2006, 34:1996-2003.
19 - Hall TS. The pathophysiology of cardiopulmonary bypass. The risks and benefits of hemodilution. Chest. 1995, 107:1125-33.
20 - Iribarren C, Jacobs DR Jr, Sidney S, et al: Cohort study of serum total cholesterol and in-hospital incidence of infectious diseases. Epidemiol Infect. 1998, 121:335-47.
21 - Almog Y, Shefer A, Novack V, et al: Prior statin therapy is associated with a decreased rate of severe sepsis. Circulation. 2004, 110:880-5.
22 - Coleman CI, Lucek DM, Hammond J, et al: Preoperative statins and infectious complications following cardiac surgery. Curr Med Res Opin. 2007, 23:1783-90.
23 - Ray KK, Cannon CP. The potential relevance of the multiple lipid-independent (pleiotropic) effects of statins in the management of acute coronary syndromes. J Am Coll Cardiol. 2005, 46:1425-33.
24 - Florens E, Salvi S, Peynet J, et al: Can statins reduce the inflammatory response to cardiopulmonary bypass? A clinical study. J Card Surg. 2001, 16:232-9.
25 - Fraunberger P, Gröne E, Gröne HJ, et al: Simvastatin reduces endotoxin-induced nuclear factor kappaB activation and mortality in guinea pigs despite lowering circulating low-density lipoprotein cholesterol. Shock. 2009, 32:159-63.
26 - Franke A, Lante W, Fackeldey V, et al: Pro-inflammatory cytokines after different kinds of cardio-thoracic surgical procedures: is what we see what we know? Eur J Cardiothorac Surg. 2005, 28:569-75.
27 - Weiland AP, Walker WE. Physiologic principles and clinical sequelae of cardiopulmonary bypass. Heart Lung. 1986, 15:34-9. 40
28 - Hill GE, Pohorecki R, Whitten CW. Plasma lipid concentrations correlate inversely with CPB-induced interleukin-6 release. Can J Anaesth. 1998, 45:509-14.
29 - Carmona F, Manso PH, Vicente WV, et al: Risk stratification in neonates and infants submitted to cardiac surgery with cardiopulmonary bypass: a multimarker approach combining inflammatory mediators, N-terminal pro-B-type natriuretic peptide and troponin I. Cytokine. 2008, 42:317-24.
30 - Giovannini I, Boldrini G, Chiarla C, et al: Pathophysiologic correlates of hypocholesterolemia in critically ill surgical patients. Intensive Care Med. 1999, 25:748-51.
31 - Dunham CM, Fealk MH, Sever WE 3rd. Following severe injury, hypocholesterolemia improves with convalescence but persists with organ failure or onset of infection. Crit Care. 2003, 7:R145-53.
32 - Bonville DA, Parker TS, Levine DM, et al: The relationships of hypocholesterolemia to cytokine concentrations and mortality in critically ill patients with systemic inflammatory response syndrome. Surg Infect (Larchmt). 2004, 5:39-49.

## Claims

1. A method for predicting sepsis onset in a patient who has undergone a surgical operation and who had a low preoperative cholesterolemia, wherein said method comprises the step of measuring the IL-8 blood level in said patient after the surgical operation, wherein an increase in IL-8 level within 6 hours after the surgical operation is associated with sepsis onset.

2. The method according to claim 1, wherein said method comprises the step of measuring the IL-8 and procalcitonin (PCT) blood levels in said patient after the surgical operation, wherein an increase in IL-8 and PCT levels within 6 hours after the surgical operation is associated with sepsis onset.

3. A method according to claim 1 or 2, wherein the surgery is bowel surgery, lung surgery, cardiac surgery, laparotomy, elective surgery, aortic aneurysm repair, or liver resection.

4. A method according to claim 1 or 2, wherein the surgery is bowel surgery, lung surgery, or cardiac surgery.

5. A method according to claim 1 or 2, wherein the surgery is cardiac surgery with cardiopulmonary bypass.

## Patentansprüche

1. Verfahren zum Vorhersagen des Ausbruchs von Sepsis in einem Patienten, der sich einer chirurgischen Operation unterzogen hat und der eine niedrige präoperative Cholesterinämie hatte, wobei das Verfahren den Schritt der Messung des Blut IL-8 Spiegels in dem Patienten nach der chirurgischen Operation umfasst, wobei eine Erhöhung des IL-8 Spiegels innerhalb von 6 Stunden nach der chirurgischen Operation mit dem Ausbruch von Sepsis assoziiert wird.

2. Verfahren gemäß Anspruch 1, wobei das Verfahren den Schritt der Messung der Blut IL-8 und Procalcitonin (PCT) Spiegel in dem Patienten nach der chirurgischen Operation umfasst, wobei eine Erhöhung der IL-8 und PCT Spiegel innerhalb von 6 Stunden nach der chirurgischen Operation mit dem Ausbruch von Sepsis assoziiert wird.

3. Verfahren gemäß Anspruch 1 oder 2, wobei die Operation eine Darmoperation, Lungenoperation, Herzoperation, Laparotomie, elektive Operation, Aortenaneurysmareparatur oder Leberresektion ist.

4. Verfahren gemäß Anspruch 1 oder 2, wobei die Operation eine Darmoperation, Lungenoperation oder Herzoperation ist.

5. Verfahren gemäß Anspruch 1 oder 2, wobei die Operation eine Herzoperation mit kardiopulmonalem Bypass ist.

## Revendications

1. Procédé pour prédire l'apparition d'une sepsie chez un patient qui a subi une opération chirurgicale et qui avait une faible cholestérolémie préopératoire, dans lequel ledit procédé comprend l'étape de mesure du taux d'IL-8 dans le sang chez ledit patient après l'opération chirurgicale, dans lequel une augmentation du taux d'IL-8 en l'espace de 6 heures après l'opération chirurgicale est associée à l'apparition d'une sepsie.

2. Procédé selon la revendication 1, dans lequel ledit procédé comprend l'étape de mesure des taux d'IL-8 et de procalcitonine (PCT) dans le sang chez ledit patient après l'opération chirurgicale, dans lequel une augmentation des taux d'IL-8 et de PCT en l'espace de 6 heures après l'opération chirurgicale est associée à l'apparition d'une sepsie.

3. Procédé selon la revendication 1 ou 2, dans lequel la chirurgie est une chirurgie de l'intestin, une chirurgie pulmonaire, une chirurgie cardiaque, une laparotomie, une chirurgie élective, la réparation d'un anévrisme aortique ou la résection du foie.

4. Procédé selon la revendication 1 ou 2, dans lequel la chirurgie est une chirurgie de l'intestin, une chirurgie pulmonaire ou une chirurgie cardiaque.

5. Procédé selon la revendication 1 ou 2, dans lequel la chirurgie est une chirurgie cardiaque avec une dérivation cardiopulmonaire.
